Europäisches Patentamt

European Patent Office

Office européen des brevets

(19)

(11) Publication number: **0 009 267**
**B1**

## (12) EUROPEAN PATENT SPECIFICATION

(45) Date of publication of patent specification: 25.11.81

(51) Int. Cl.³: **C 07 C 83/00**

(21) Application number: **79200454.1**

(22) Date of filing: **15.08.79**

(54) Process for the preparation of p-nitroso-diphenylhydroxylamines.

(30) Priority: **18.08.78 GB 3379078**

(43) Date of publication of application:
**02.04.80 Bulletin 80/7**

(45) Publication of the grant of the European patent:
**25.11.81 Bulletin 81/47**

(84) Designated Contracting States:
**BE DE FR GB**

(56) References cited:
**DE - A - 2 020 043**
**DE - B1 - 2 703 919**

(73) Proprietor: **Akzo N.V.**
**IJssellaan 82**
**NL-6826 DW Arnhem (NL)**

(72) Inventor: **Battey, Paul Kenneth**
**4 Ellerbrook Drive, Lathom**
**Ormskirk, Lancs. (GB)**
Inventor: **Hope, Peter**
**47 Paradise Lane**
**Formby, Liverpool (GB)**
Inventor: **Bergfeld, Manfred**
**Dürerstrasse 1**
**Erlenbach (DE)**

(74) Representative: **Sieders, René et al,**
**P.O. Box 314**
**NL-6800 AH Arnhem (NL)**

Courier Press, Leamington Spa, England.

## Process for the preparation of p-nitroso-diphenyl-hydroxylamines

The present invention relates to a processor for the preparation of p-nitroso-diphenyl-hydroxylamines.

p-Nitroso-diphenyl-hydroxylamines may be obtained by the dimerisation of nitroso benzenes in concentrated sulphuric acid (E. Bamberger et al., B. *31*, page 1513 (1898). since the process is exothermic, comparatively large quantities of concentrated sulphuric acid must be used to achieve adequate mixing of the components and removal of heat. Ice or water must be added to the mixture to isolate the reaction product. The large quantities of dilute sulphuric acid thereby formed are neutralized and subsequently removed as effluent, since the recovery of the sulphuric acid is impracticable on the grounds of cost, partly because it is contaminated with nitrogen-containing organic material. The process is therefore unsuitable for the industrial manufacture of p-nitroso-diphenyl-hydroxylamines.

In the process described in DT—OS No. 2,020,043, the dimerizing rearrangement of nitroso benzenes is carried out with a sulphuric acid having a concentration of at least 50% by weight, preferably at least 75% by weight, in the presence of an organic liquid, e.g. an aliphatic hydrocarbon, a halogenated hydrocarbon or an aromatic nitro compound, at temperatures of from 5 to 50°C. Since the reaction product decomposes very rapidly in the strongly acid solution at higher temperatures, the heat of reaction must be rapidly removed. This function is performed by the organic liquid. However, in this process sulphuric acid must also be used in large excess, namely in up to ten times, preferably 2.5 to 6.5 times the molar quantity of nitroso benzene. This again gives rise to the disadvantages already described in connection with the process of Bamberger et al. Moreover, the reaction product obtained is of poor quality. Since it contains considerable quantities of tarry constituents in addition to products which are sulphonated in the nucleus. When stoichiometric quantities of sulphuric acid are used, p-nitroso-diphenylhydroxylamine precipitates in the form of its sulphate as a viscous, dark mass which is technically difficult to handle and also contains substantial quantities of tar in addition to products which are sulphonated in the nucleus.

In the process described in DE 1,147,237, hydrogen fluoride is used as a dimerizing agent instead of concentrated sulphuric acid. The reaction is carried out at temperatures of between −20°C and 50°C, optionally in the presence of an inert organic solvent (see K. Wiechert et al.), Z. Chem. *15* (1955) page 21). In this process also, the dimerizing agent is used in a large excess because hydrogen fluoride serves not only as the catalyst but also as the solvent. If hydrogen fluoride is used in the stoichiometric amount, a yield of only 25% of the theoretical yield is obtained and the product is contaminated with derivatives which are fluorinated in the nucleus. The hydrogen fluoride is distilled off under vacuum after the reaction and may be recycled. However, hydrogen fluoride has the disadvantage of having a low boiling point and very pungent odour and its vapours are highly toxic to the respiratory system. Moreover, the desired product is obtained as a viscous mass still contaminated with about 10 to 20% of hydrogen fluoride adhering to it. This hydrogen fluoride is virtually impossible to recover because under the conditions which would be necessary for this, p-nitroso-diphenyl-hydroxylammonium fluorides decompose to a blackish brown mass. The wash water is therefore also contaminated with hydrogen fluoride and problems of corrosion also arise. The process is therefore very expensive in the apparatus required, so that it also is hardly suitable for the production of p-nitroso-diphenyl hydroxylamines on an industrial scale.

It is known that p-nitroso-diphenyl hydroxylamine is formed together with nitrobenzene when nitrosobenzene is treated with peroxy trifluoro acetic acid (J.H. Beyer, J. Org. Chem. *24*, 2038 (1959)). In this process, peroxy trifluoro acetic acid oxidizes nitrosobenzene to nitrobenzene and at the same time catalysts the dimerization of nitrosobenzene to p-nitroso-diphenyl hydroxylamine. The formation of nitrobenzene is favoured at higher temperature and the formation of p-nitroso-diphenyl hydroxylamine at lower temperatures. In the most favourable case, the quantity of p-nitroso-diphenyl hydroxylamine obtained is 35% of the theoretical quantity. The process is therefore not highly selective and again unsuitable for the commercial production of p-nitroso-diphenyl hydroxylamines.

In DE—OS 2.703.919 a process has been described for the preparation of p-nitroso-diphenyl hydroxylamines by the reaction of a nitroso compound of the benzene series on its own or with another such compound in the presence of an acid as catalyst, characterized in that an aliphatic, cycloaliphatic or aromatic sulphonic acid having a $pK_a$ value $\leqslant$ 1, perchloric acid or trifluoro acetic acid is used as the catalyst in a quantity of at least 0.5 mol per mol of nitroso compound(s), and the reaction is carried out at a temperature of from −20°C to 60°C.

According to Houben-Weyl: Methoden der organischen Chemie, Bd.IV (1955), page 67, hydrogen fluoride is a member of a group collectively called Lewis acids that generally include halides of a variety of metals such as zinc, boron, titanium, tin, antimony, iron, aluminium, bismuth and zirconium.

It has now surprisingly been found that, unlike hydrogen fluoride, certain Lewis acids are very useful catalysts for dimerizing nitrosobenzenes.

According to the invention subject process for the preparation of p-nitroso-diphenyl hydroxylamines is characterized in that the Lewis acid catalyst is boron trifluoride, a complex thereof, or ferric chloride together with a carboxylic acid.

When using a complex of boron trifluoride it is preferred to take its commonly used complexes with e.g. ether, methanol and acetic acid. The ferric chloride and the carboxylic acid are generally used in a ratio of at least 1.0 mol of ferric chloride and 1.0 mol of the carboxylic acid per mol of the nitroso compound. Preferred carboxylic acids are formic acid and acetic acid.

The process according to the invention is particularly suitable for the preparation of p-nitroso-diphenyl hydroxylamine by dimerizing rearrangement of nitrosobenzene but it is also suitable for the preparation of asymmetrically substituted p-nitroso-diphenyl hydroxylamines, which are obtained by dimerizing rearrangement of ortho- or meta- substituted nitrosobenzenes or by the reaction of a parasubstituted nitrosobenzene with anitrosobenzene which is unsubstituted in the para-position. The starting compounds used may be any nitroso compounds of the benzene series whose substituents are inert towards the catalyst. Examples of such substituents include nitro, alkoxy, haloalkyl, alkyl and carbalkoxy groups. The nitroso compounds may be substituted with one or more substituents. The following starting materials are preferred:

o-nitrosotoluene, o-chloro-nitrosobenzene, m-chloro-nitrosobenzene, o-methoxy-nitrosobenzene, o-nitro-nitrosobenzene, m-trifluoro-methylnitrosobenzene, 2,6-dichloro-nitrosobenzene, 2,6-dimethyl-nitrosobenzene, m-fluoro-nitrosobenzene, o-methyl-nitrosobenzene, 2,5-dichloro-nitrosobenzene, m-nitro-nitrosobenzene and 2-nitrosobenzoic acid methyl ester.

Mixtures of two or more of the above mentioned starting materials may, of course, be used.

The process of the present invention is attractive from a technical and commercial viewpoint because:

(a) use is made of economical inorganic catalysts which are simple to separate from the organic phase,

(b) the use of present Lewis acid catalysts shows little tendency to byproduct formation, and

(c) the process can be carried out under a variety of temperature/time conditions.

The quantity of catalyst to be used depends to a certain extent on the nature of the Lewis acid but for obtaining high yields it is preferred to use the catalyst in at least the stoichiometric ratio, i.e. in quantities corresponding to at least 0.5 mol per mol of nitroso compound. The general range for the quantity of catalyst is from 0.5 to 10 mol per mol of nitroso compound, but it is a particular advantage of the catalysts to be used according to the invention that they may be used in smaller quantities than the conventional acids, i.e. preferably in quantities of form 0.5 to 1.0 mol per mol of nitroso compound. This not only has economical advantages but also considerably facilitates working up of the reaction mixture.

The process according to the invention may be carried out in the presence of an organic solvent.

If an organic solvent is used, it is not absolutely necessary to operate in the homogeneous phase but both the nitroso compound and the catalyst should be at least partly soluble in the solvent. It is also desirable for the resulting salt of p-nitroso-diphenyl hydroxylamine to be soluble in the solvent because the reaction product can then be obtained in a very pure form. Suitable solvents include aliphatic, cycloaliphatic and aromatic hydrocarbons, and these may carry one more or more alkyl, halogen and/or nitro groups. The solvents preferably used are nitromethane, nitrobenzene, methylenechloride, chloroform, 1,2-dichloroethane, 1,1,1-trichloromethane and 1,1,2,2-tetrachloroethane. The process according to the invention is generally carried out at temperatures of from $-20°C$ to $60°C$, preferably from $20°C$ to $40°C$. The reaction is strongly exothermic and therefore requires careful cooling of the reaction mixture, even when no solvent is used.

The process according to the invention may suitably be carried out by introducing a solution or suspension of the nitroso compound(s) in an organic solvent into the reaction vessel and then adding the catalyst portionwise with vigorous stirring and with cooling. It is advantageous to carry out the reaction in a low boiling solvent such as methylene chloride under reflux. In that case, the heat of reaction is easily removed by cooling due to evaporation.

The nitroso-diphenyl-hydroxylamines are obtained in a highly pure state by the process according to the invention but if necessary, they may be further purified in known manner, for example by recrystallisation or reprecipitation. This may be carried out by first dissolving the crude product in an aqueous alkali metal or alkaline earth metal hydroxide or in sodium sulphite and then reprecipitating it by the addition of a mineral acid.

Nitro-diphenyl-hydroxylamines are valuable compounds which may be worked up, for example, into antioxydants, antiozonants or dyes. Especially they are valuable as intermediates for the manufacture of p-phenylenediamine antidegradants which are widely used in the rubber industry.

The present invention will be further described with reference to the following Examples.

### Example 1

Nitrosobenzene (0.025 mol, 2.68 g) was dissolved in methylene dichloride (6 cm³) and a solution of boron trifluoride etherate (0.0275 mol, 3.91 g) in methylene dichloride (4 cm³) added rapidly to the stirred green coloured solution. A mildly exothermic reaction ensued resulting in gentle reflux of the methylene dichloride and the formation of a dark red/brown solution. After a further period of 15 minutes stirring, this solution was added to distilled water (75 cm³) in a 1 litre vessel and the methylene dichloride distilled out under water pump vacuum on a rotary evaporator (35°C/*ca* 2.7 kPa). The precipitated N-phenyl-(4-nitrosophenyl)-hydroxylamine was filtered off, washed with ice-cold water (3 × 10 cm³) and dried (50°C/0.13 kPa) to constant weight.

Yield = 2.56 g (96%) of a fine brown powder.

### Example 2

Nitrosobenzene (0.025 mol, 2.68 g) was dissolved in methylene dichloride (6 cm³) and a solution of boron trifluoride etherate (0.0138 mol, 1,96 g) in methylene dichloride (4 cm³) added rapidly to the stirred green coloured sloution. A mildly exothermic reaction ensued, causing the methylene dichloride to reflux very gently and resulted in a dark red/brown solution. After a further period of 15 minutes stirring this solution was added to distilled water (75 cm³) in a litre vessel and the methylene dichloride distilled out under water pump vacuum on a rotary evaporator (35°C/*ca* 2.7 kPa). The precipitated N-phenyl-(4-nitrosophenyl)-hydroxylamine was filtered off, washed with ice-cold water (3 × 10 cm³) and dried (50°C/0.13 kPa) to constant weight.

Yield = 2.47 g (92%) of a fine orange/brown solid.

### Example 3

Nitrosobenzene (0.025 mol, 2.68 g) and anhydrous ferric chloride (0.025 mol, 4.05 g) were mixed and stirred whilst a solution of formic acid (0.025 mol, 1.15 g) in methylene dichloride (10 cm³) was added rapidly. An exothermic reaction ensued which caused the methylene dichloride to reflux and resulted in a deep orange/brown solution and orange/brown tar. After stirring for a further 15 minutes the total reaction mixture was dissolved in methanol. Quantitative UV indicates a quantative conversion to N-phenyl-(4-nitrosophenyl)-hydroxylamine.

### Claims

1. A process for the preparation of p-nitroso-diphenyl hydroxylamines by the reaction of at least one nitroso compound of the benzene series in the presence of a Lewis acid as a catalyst in an amount of at least 0.5 mole per mole of the nitroso compound at a temperature in the range of −20°C to 60°C, characterized in that the Lewis acid is boron trifluoride, a complex thereof, or ferric chloride together with a carboxylic acid.

2. A process as claimed in claim 1 wherein the boron tri-fluoride complex is a complex with ether.

3. A process as claimed in claim 1 wherein ferric chloride and the carboxylic acid are used in a ratio of at least 1.0 mole of ferric chloride and 1.0 mole of the carboxylic acid per mole of the nitroso compound.

4. A process as claimed in claim 1 or 3 wherein ferric chloride is used together with formic or acetic acid.

5. A process as claimed in any of the preceding claims wherein the reaction is carried out at a temperature in the range of 20°C to 40°C.

6. A process as claimed in any of the preceding claims wherein the reaction is carried out by adding the catalyst portionwise to a solution or suspension of the nitroso compound(s) with vigorous stirring and cooling.

7. A process as claimed in claim 6 which is carried out in methylene chloride under reflux.

### Revendications

1. Procédé pour préparer des p-nitroso-diphénylhydroxylamines par réaction d'au moins un composé nitrosé de la série benzène en présence d'un acide Lewis comme catalyseur dans une quantité d'au moins 0,5 mole par mole du composé nitrosé à une température située dans la gamme comprise entre −20 et 60°C, caractérisé en ce que l'acide Lewis est le trifluorure de bore, un complexe de celui-ci ou le chlorure ferrique en combinaison avec un acide carboxylique.

2. Procédé selon la revendication 1, caractérisé en ce que le complexe de trifluorure de bore est un complexe avec l'éther.

3. Procédé selon la revendication 1, caractérisé en ce que le chlorure ferrique et l'acide carboxylique sont utilisés dans un rapport d'au moins 1,0 mole de chlorure ferrique et de 1.0 mole d'acide carboxylique par mole de composé nitrosé.

4. Procédé selon l'une quelconque des revendications 1 et 3, caractérisé en ce que le chlorure ferrique est utilisé en combinaison avec l'acid formique ou acétique.

5. Procédé selon l'une quelconque des revendications précédentes, caractérisé en ce que la réaction est effectuée à une température située dans la gamme comprise entre 20 et 40°C.

6. Procédé selon l'une quelconque des revendications précédentes, caractérisé en ce que la réaction est effectuée en ajoutant le catalyseur par portions à une solution ou suspension du (des) composé(s) sous forte agitation et refroidissement.

7. Procédé selon la revendication 6, caractérise en ce qu'il est effectué sous reflux dans du chlorure de méthylène.

## Patentansprüche

1. Verfahren zur Herstellung von p-Nitrosodiphenylhydroxylaminen durch Umsetzung von mindestens einer Nitrosoverbindung der Benzolreihe in Anwesenheit einer Lewis-Säure als Katalysator in einer Menge von mindestens 0,5 Mol pro Mol der Nitrosoverbindung bei einer Temperatur im Bereich von −20°C bis 60°C, dadurch gekennzeichnet, daß es sich bei der Lewis-Säure um Bortrifluorid, einen Bortrifluorid-Komplex oder um Eisen (III)-chlorid in Kombination mit einer Carboxylsäure haldelt.

2. Verfahren gemäß Anspruch 1, dadurch gekennzeichnet, daß es sich bei Bortrifluorid-Komplex um einen Komplex mit Ather handelt.

3. Verfahren gemäß Anspruch 1, dadurch gekennzeichnet, daß das Eisen (III)-chlorid und die Carboxylsäure im Verhältnis von mindestens 1,0 Mol Eisen (III)-chlorid und 1,0 Mol Carboxylsäure pro Mol der Nitrosoverbindung verwendet werden.

4. Verfahren gemäß Anspruch 1 oder 3, dadurch gekennzeichnet, daß Eisen (III)-chlorid zusammen mit Ameisen- oder Essigsäure verwendet wird.

5. Verfahren gemäß Anspruch 1—4, dadurch gekennzeichnet, daß die Umsetzung bei einer Temperatur im Bereich von 20°C bis 40C erfolgt.

6. Verfahren gemäß Anspruch 1—5, dadurch gekennzeichnet, daß man die Umsetzung durchführt, indem man den Katalysator einer Lösung oder Suspension der Nitrosoverbindung(en) portionsweise unter kräftigem Rühren und Kühlen zusetzt.

7. Verfahren gemäß Anspruch 6, dadurch gekennzeichnet, daß die Umsetzung in Methylenchlorid unter Rückfluß erfolgt.